(19) Europäisches Patentamt
European Patent Office
Office européen des brevets

(11) Publication number: **0 058 442**
**B1**

(12) **EUROPEAN PATENT SPECIFICATION**

(45) Date of publication of patent specification: **02.01.85**

(51) Int. Cl.⁴: **C 07 C 69/593,** C 07 C 53/00, C 07 C 67/36, C 07 C 51/083

(21) Application number: **82200080.8**

(22) Date of filing: **22.01.82**

(54) **A process for the co-production of an alkylidene dicarboxylate and a carboxylic acid.**

(30) Priority: **09.02.81 GB 8103896**

(43) Date of publication of application:
**25.08.82 Bulletin 82/34**

(45) Publication of the grant of the patent:
**02.01.85 Bulletin 85/01**

(84) Designated Contracting States:
**DE FR GB IT**

(56) References cited:
**EP-A-0 031 606**
**US-A-3 579 566**
**US-A-4 102 920**
**US-A-4 218 340**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Drent, Eit**
**Badhuisweg 3**
**NL-1031 CM Amsterdam (NL)**

(74) Representative: **Puister, Antonius Tonnis, Mr. et al**
**P.O. Box 302**
**NL-2501 CH The Hague (NL)**

**EP 0 058 442 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for the co-production of an alkylidene dicarboxylate and a carboxylic acid.

US Patent Specification No. 3,579,566 discloses that the hydrogenation of a carboxylic acid anhydride to give an alkylidene dicarboxylate plus an acid may be carried out using a catalyst comprising a complex of a Group VIII noble metal with a trihydrocarbyl phosphine, arsine or stibine. However, the reaction products obtained by this process are complex mixtures and the yield of the desired dicarboxylate is very low. In particular, the yield of carboxylic acid which, according to the stoichiometry of the reaction should be formed in a quantity equimolar with the dicarboxylate, is formed in large excess. Thus in Example 1 of said specification, the molar ratio of acetic acid to ethylidene diacetate is 6.7:1. Further, the temperatures and pressures required for the reaction are very high.

UK Application No. 2,034,307 discloses that this hydrogenation process can be improved somewhat by carrying out the reaction using an insoluble metal hydrogenation catalyst, such as palladium on charcoal in the presence of a strong protonic acid. However, the molar ratio of acetic acid to ethylidene diacetate produced is in most cases much greater than one, and the yields obtained are generally low. In particular, the results obtained when using HCl, HBr or HI as the strong acid, are markedly inferior to the results obtained using other acids, giving very poor yields and selectivities. A further major disadvantage of this process is that it requires a hydrogen stream which is substantially free from carbon monoxide gas. Despite the statement at lines 14 to 16 page 2 of said specification, the presence of even small quantities of carbon monoxide dramatically reduces both the yield and the selectivity of the reaction (see comparative examples 14 and 15 herein). This is a disadvantage since a convenient source of hydrogen available to industry is that present in synthesis gases, which are carbon monoxide/hydrogen mixtures.

UK Patent Specification No. 1,538,782, discloses that ethylidene diacetate can be prepared by reacting methyl acetate and/or dimethyl ether, with carbon monoxide and hydrogen, in the presence of a group VIII noble metal catalyst and a source of halide. Large quantities of acetic anhydride are formed as by-product. The following passage occurs in the specification:

"The mechanism of the reaction or reactions occurring is not known. It is unlikely, however, that the desired ethylidene diacetate is primarily formed by reaction (i.e., reduction) of acetic anhydride with hydrogen present in the system, a reaction disclosed by Fenton in US Patent No. 3,579,566, because of the behaviour of the reaction system of this invention in the presence of especially preferred catalyst systems. According to recognized reaction mechanism postulates with such preferred catalyst systems (see Khan and Martell, "Homogeneous Catalysts of Metal Complexes", Vol. I, Academic Press, New York (1974) at pages 49 and 315), the formation of organo-metallic complexes of such catalysts with acid anhydrides would be expected to be far less favored than would the formation of such complexes with suspected reaction intermediates such as acyl halides. The ready formation of such complexes with acyl halides leads to facile reduction of the acyl halide in comparison to the acid anhydrides which would tend to preclude involvement of anhydride reduction as a significant factor in the observed ethylidene diacetate formation reaction".

This reasoning is indeed persuasive. The concentrations of acetic anhydride produced in the process are large, and it would be expected that if hydrogenation of acetic anhydride could take place under the reaction conditions, it would do so and the acetic anhydride would be converted to ethylidene diacetate plus acetic acid *in situ*. Thus the acetic anhydride appears to be undesired by-product formed from one of the reaction intermediates, rather than an intermediate itself.

It has now been found that carboxylic acid anhydrides can be used as feedstock in a process for the co-production of alkylidene dicarboxylates and carboxylic acids which process gives very high selectivity to the desired products.

The invention therefore provides a process for the co-production of an alkylidene dicarboxylate and a carboxylic acid, characterized in that a carboxylic acid anhydride is hydrogenated in the presence of carbon monoxide and a homogeneous Group VIII metal catalyst together with a chloride, bromide or iodide in addition to any halide being present in the Group VIII metal catalyst and in the additional presence of a promotor comprising an organo oxygen, nitrogen, phosphorus, arsenic or antimony compound having a lone pair of electrons.

A wide range of carboxylic acid anhydrides can be used as feedstock in the process according to the invention. In general terms, the reaction proceeds according to the equation:

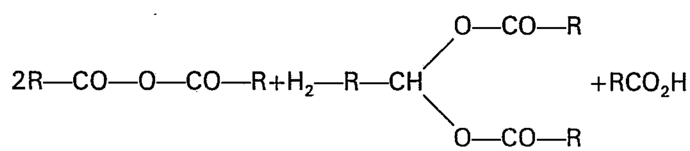

Preferably a symmetrical anhydride is used as feedstock so that only a single dicarboxylate and a single carboxylic acid are produced. However, the reduction can if desired by carried out using a mixed

anhydride (i.e., the R's are different) in which case a mixture of products is obtained. In certain circumstances, the groups R in the product may differ from the groups R in the anhydride. For example, if an R in the anhydride contains an olefinic double bond, this will be hydrogenated under the reaction conditions and products containing the corresponding saturated R group will be obtained.

Preferably, each R in the anhydride independently represents an alkyl, alkenyl, alkynyl or aryl group, which may if desired be substituted by one or more substituents, for example halogen atoms, phenyl groups and alkoxy groups, but are preferably unsubstituted, and preferably have up to 20 carbon atoms. More preferably each R represents an unsubstituted alkyl group having up to 6 carbon atoms. Acetic anhydride is an especially preferred feedstock, in which case the reaction products are ethylidene diacetate and acetic acid.

Preferably the group VIII metal used as catalyst is selected from cobalt, nickel, rhodium, palladium, osmium, iridium and platinum, with the use of palladium and, especially, rhodium and nickel being preferred. The metal may for example be added in the form of a salt with a mineral acid, for example a halide, nitrate or sulphate, or with an organic acid, for example a carboxylate having up to 20 carbon atoms, especially an alkanoate, such as an acetate. Alternatively, the metal may be in zero valent form complexed by ligands such as the phosphine ligands described below, carbon monoxide, or acetylacetonates. The catalyst must be homogeneous with the reaction medium, and the presence of carbon monoxide is necessary in order to stabilize the catalyst in solution. The carbon monoxide is suitably provided in admixture with the hydrogen gas supplied to the system. The relative amounts of hydrogen and carbon monoxide supplied to the system may vary over a very wide range. For example, a molar ratio $H_2$:CO in the range 1:99 to 99:1 is suitable, with a molar ratio of 25:75 to 95:5 being preferred.

The quantity of catalyst present in the system is generally determined by economic considerations. Quantities of catalyst of between 0.01 to 10, especially 0.05 to 5, mole % based on the number of moles of anhydride used as feedstock, are generally suitable.

The process according to the invention is carried out in the presence of a halide selected from chloride bromide or iodide. The use of a bromide or, especially, an iodide, is preferred. The halide used is in addition to any halide which may be present in the catalyst or catalyst precursor.

The halide is preferably added in the form of an alkyl halide, for example methyl iodide, an acyl halide, for example acetyl bromide, a hydrogen halide, elemental halogen, or a halide salt of an alkali or alkaline earth metal, for example lithium on sodium iodide. The use of an alkyl halide is preferred.

When using a symmetrical anhydride of formula $(RCO)_2O$ as feedstock and an organic halide, it may be convenient in order to minimize the formation of mixed products, to select the organic halide having the formula R.Halide or R.CO.Halide. Thus for example when using acetic anhydride as feedstock, methyl iodide or acetyl bromide are suitable halides, whereas when using propionic anhydride as feedstock, it may be more convenient to use ethyl iodide or propionyl bromide.

The quantity of halide added may vary over a wide range, and generally, the rate of the reaction increases with the concentration of halide. For example, the halide may be present in an amount of up to 10 molar percent based on the number of moles of anhydride used as feedstock. Preferably, the molar ratio of added halide to metal catalyst is in the range of from 1:1 to 200:1, especially 5:1 to 50:1, the halide calculated as halide ion and the catalyst calculated as atomic group VIII metal. These quantities of halide are of course in addition to any halide which may be present in the catalyst or catalyst precursor.

As stated above, the hydrogen gas stream supplied to the system may contain carbon monoxide. It may also if desired contain inert gases, such as nitrogen. The reaction is suitably carried out under a pressure of from 10 to 150 bars, especially 20 to 100 bars. Higher pressures, for example up to 1000 bars, can of course be used, but are generally undesirable for economic reasons.

The reaction temperature is suitably in the range of from 100 to 200°C, especially 130 to 170°C.

The reaction is suitably carried out under substantially anhydrous conditions to prevent hydrolysis of the starting material and the product. However, the presence of the minor amounts of water normally found in the commercial forms of the components of the reaction mixture presents no problem.

The process according to the invention is often conveniently carried out using excess carboxylic acid anhydride as solvent. However, if desired, any suitable additional solvent may be used. Suitable inert solvents include hydrocarbons, for example xylene or hexane, ethers, for example tetrahydrofuran, amides, for example dimethylformamide, nitriles, for example acetonitrile, and sulphur-containing compounds, for example sulpholane. The use of solvents which are reactive under the reaction conditions is of course undesirable.

Suitable promotors are organo oxygen, nitrogen, phosphorus, arsenic and antimony compounds having a lone pair of electrons. Preferred promotors are organo nitrogen or, especially, organo phosphorus compounds, especially tri-organo phosphines.

Suitable oxygen-containing promoters include compounds containing a hydroxy, carbonyl, carbonyloxy or ether group. Typical compounds of this type include carboxylic acids, especially hydroxy or alkoxy substituted acids, such as methoxyacetic acid or hydroxyacetic acid, ethers such as tetrahydrofuran, and amides, such as dimethylacetamide. Amides are of course an example of a

promotor containing both nitrogen and oxygen atoms. It may be suitable to use an oxygen-containing compound as a solvent which may also have a promoting effect.

Suitable phosphorus, antimony and arsenic promoters include those of the general formula XR′R″R‴, in which X represents phosphorus, antimony or arsenic, and each of R′, R″ and R‴ independently represents an optionally substituted alkyl, cycloalkyl or aryl group, or R′ has this meaning and R″ and R‴ together represent an alkylene group. Optional substituents may be any moieties inert under the reaction conditions, for example halogen atoms, alkoxy groups and phenyl groups. Preferably however R′, R″ and R‴ are hydrocarbyl groups. Preferably any alkyl group has up to 20 carbon atoms; any cycloalkyl group has up to 7 carbon atoms; any aryl group is a phenyl group; and any alkylene group has up to 20 carbon atoms.

Especially preferred promotors of this type are those in which each of R′, R″ and R‴ independently represents an alkyl group or a phenyl group. For economic reasons, it is generally preferred that each of R′, R″ and R‴ represents the same group.

Preferably X represents a phosphorus atom. Typical phosphine promotors are trimethylphosphine, triethylphosphine, tributylphosphine and triphenylphosphine.

Suitable nitrogen-containing promotors include those of the general formula NR′R″R‴ where R′, R″ and R‴ have the meanings given above, and also compounds in which the nitrogen atom forms part of a heterocyclic ring. Typical promotors of this type include pyrrole, pyrrolidine, pyridine, piperidine, pyrimidine, picoline and quinoline, and substituted analogues thereof, e.g. alkyl-substituted analogues.

The amount of promotor used is not critical. Except in those cases where the promotor used may also be employed as a solvent, the ratio of promotor to catalyst is preferably in the range of from 1:1 to 20:1, especially 2:1 to 10:1, calculated as moles of promotor per gram atom of group VIII metal.

In a preferred embodiment of the process according to the invention the carboxylic acid anhydride used as feedstock has been prepared in a separate reaction step by the carbonylation of an ester in the presence of a suitable catalyst. Suitable methods for carrying out this carbonylation are described for example in UK Patent Specifications 1523346 and 1468940.

For example, the ester may be carbonylated in the presence of a rhodium containing catalyst together with a halide. Any of the halides described hereinabove for the hydrogenation of an anhydride, may be used, but preferably the halide is a bromide or iodide of an alkali metal. Lithium iodide is especially suitable. Surprisingly, it has been found that the rate of carbonylation is much increased by the addition of small quantities of either water or carboxylic acid to the reaction mixture. Suitably, the molar ratio of water or carboxylic acid to halide is in the range of from 1:1 to 1:10, especially 1:1.5 to 1:5. The carboxylic acid added is preferably the acid or one of the acids corresponding to the anhydride being prepared, in order to avoid the production of undesired mixed products. If water is added, it may be added as such, or, in suitable cases, as water of crystallization of the halide: the commercial forms of lithium iodide contain from 2 to 3 moles of water of crystallization per mole of LiI, and this quantity of water is adequate to enhance the rate of the carbonylation reaction.

It has also been found that when using metal halides as promotors in the carbonylation reaction, the rate of reaction can be increased by the addition of a suitable crown ether capable of complexing the metal ion. Thus for example when using sodium iodide as the halide, the addition of 15-crown-5 increases the rate of the reaction. When the reaction mixture contains both a crown ether and water or a carboxylic acid, the promoting effect is particularly marked.

The carbonylation reaction is suitably carried out at a temperature in the range of from 130 to 220°C, especially 160 to 200°C. Generally, the optimum temperature for carbonylation for an ester is somewhat higher than the optimum temperature for hydrogenation of the anhydride thus produced.

The ester carbonylation reaction is preferably carried out using a carbon monoxide gas stream which does not contain large quantities of hydrogen, although quantities of hydrogen of for example up to about 15% by volume of the gas stream, can be tolerated; above this leverl, the production of by-products tends to increase.

A stream of carbon monoxide free from large quantities of hydrogen can be obtained by carrying out the hydrogenation process according to the invention using a mixed carbon monoxide/hydrogen stream, (i.e. a synthesis gas stream), suitably maintaining the initial charge of carboxylic acid anhydride in excess of that required to react with the hydrogen. The resulting carbon monoxide can then be recycled to the ester carbonylation step, to produce a further charge of anhydride. This two-step process for the co-production of an alkylidene dicarboxylate and a carboxylic acid from an ester is ideally suited to the utilization of industrial synthesis gases containing carbon monoxide and hydrogen in a molar ratio of 2:1.

The anhydride product of the carbonylation step may if desired be separated from the other components of the reaction mixture from that step before being hydrogenated by the process according to the invention. Conventional techniques such as distillation may be used. However, complete separation is not essential. For example, amounts of ester in the anhydride feedstock for the process according to the invention, of up to about 1 mole of ester per mole of anhydride, may be tolerated if desired. However, it is preferred that the feedstock should be free from substantial amounts of ester, for example greater than 10 molar percent, in order to avoid the formation of by-products and inhibition of the hydrogenation of the anhydride.

The ester to be carbonylated may be prepared by any suitable method, typically by esterification of an acid with an alcohol by known methods. The acid may be the same acid as is co-produced along with a dicarboxylate, in the process according to the invention, thus effectively recycling this acid. Alternatively, the ester may be prepared *in situ* by the carbonylation of an ether or alcohol using known techniques.

The alkylidene dicarboxylate produced by the process according to the invention may be converted into a number of other valuable products by known methods, for example into olefinic esters, or into aldehydes. For example, ethylidene diacetate, which is the preferred compound produced by the process according to the invention, can be converted into either vinyl acetate or acetaldehyde by well known methods.

Thus for example in an integrated reaction scheme, methanol, carbon monoxide and hydrogen can be converted into vinyl acetate:

$$2CH_3OH+2CH_3CO_2H \rightarrow 2CH_3CO_2CH_3+2H_2O$$

$$2CH_3CO_2CH_3+2CO \rightarrow 2(CH_3CO)_2O$$

$$2(CH_3CO)_2O+H_2 \rightarrow CH_3CH(OCOCH_3)_2+CH_3CO_2H$$

$$CH_3CH(OCOCH_3)_2 \rightarrow CH_3CO_2CH{=}CH_2+CH_3CO_2H$$

$$2CH_3OH+2CO+H_2 \rightarrow CH_3CO_2CH{=}CH_2+2H_2O.$$

Alternatively, the ethylidene diacetate can be converted into acetaldehyde:

$$CH_3CH(OCOCH_3)_2 \rightarrow CH_3CHO+(CH_3CO)_2O$$

and the overall reaction becomes:

$$CH_3OH+CO+H_2 \rightarrow CH_3CHO+H_2O.$$

The following Examples illustrate the invention.

Examples 1 to 9

All these examples were carried out using the same technique. A Hastelloy C (Trade Mark) 300 ml magnet-driven autoclave was charged with 50 mls acetic anhydride and the necessary catalyst components, flushed with carbon monoxide, and then pressurized to 40 bars with a 1:1 molar mixture of carbon monoxide and hydrogen. The autoclave was then maintained at the desired temperature for 15 hours. The contents were cooled and analysed using gas-liquid chromatography.

The results obtained are given in Table 1.

TABLE 1

| Example No. | Temp., °C | Catalyst components. (mmol) | Conversion of anhydride to products, % | Molar ratio acetic acid to ethylidene diacetate |
|---|---|---|---|---|
| 1 (comparison) | 135 | $RhCl_3.3H_2O$ (0.5)<br>$CH_3I$ (30) | 60 | 1.6:1 |
| 2 | 150 | $RhCl_3.3H_2O$(1.0)<br>$CH_3I$ (30)<br>$P(phenyl)_3$(2.5) | 60 | 1.1:1 |
| 3 | 150 | $RhCl_3.3H_2O$ (0.5)<br>$CH_3I$ (30)<br>$P(phenyl)_3$ (3.8) | 70 | 1.0:1 |
| 4 | 145 | $RhCl_3.3H_2O$ (1)<br>$CH_3I$ (30)<br>alpha-picoline (3) | 60 | 1.2:1 |
| 5 | 135 | $RhCl_3.3H_2O$ (0.5)<br>$CH_3I$ (30)<br>$P(n\text{-}butyl)_3$ | 25 | 1.1:1 |
| 6 | 135 | $RhCl_3.3H_2O$ (1.0)<br>$CH_3.CO.Cl$ (30)<br>$P(phenyl)_3$ (3.0) | 15 | 1.0:1 |
| 7 | 150 | $NiCl_2.2H_2O$ (1)<br>$CH_3I$ (30)<br>$P(phenyl)_3$ (2.5) | 75 | 1.1:1 |
| 8 | 150 | $Pd(CH_3CO_2)_2$ (1)<br>$CH_3I$ (15)<br>$P(phenyl)_3$ (3.0) | 25 | 1.0:1 |
| 9 | 150 | $Co_2(CO)_8$ (0.5)<br>$CH_3I$ (15)<br>$P(phenyl)_3$ (3.0) | 5 | 1.2:1 |

In all of Examples 1 to 9, only trace amounts of products other than acetic acid and ethylidene diacetate were observed. Examples 8 and 9 show that palladium and cobalt catalysts are less active than rhodium and nickel catalysts, but that they are still extremely selective in the production of the desired products.

Example 10 (comparison)

Example 4 was repeated exactly except that the methyl iodide was omitted. The conversion of acetic anhydride to products was less than 5%, and the molar ratio of acetic acid to ethylidene diacetate was greater than 6:1.

Example 11

The method of Examples 1 to 9 was repeated except that the autoclave was pressurised to 45 bars with a 2:1 mixture of carbon monoxide:hydrogen. The catalyst was $RhCl_3.3H_2O$ (0.5 mmol), $CH_3I$ (30 mmol) and triphenylphosphine (3.8 mmol). The temperature was 150°C. After the completion of the reaction time, 40% of the anhydride had been converted to the desired products, the molar ratio of acetic acid to ethylidene diacetate being 1.2:1.

Example 12 (comparison)

Example 11 was repeated exactly except that the methyl iodide was omitted. The conversion of anhydride to products was only 10%, and the molar ratio of acetic acid to ethylidene diacetate was 2:1.

Example 13

The procedure of Examples 1 to 9 was repeated, except that the acetic anhydride was replaced with 50 mls propionic anhydride. The catalyst used was $RhCl_3.3H_2O$ (1 mmol), triphenylphosphine (2.5

mmol) and $CH_3I$ (30 mmol). The reaction temperature was 135°C. At the end of the reaction time, 40% of the anhydride had been converted into the desired products, and the molar ratio of the dipropionate, $C_2H_5.CH.(O.CO.C_2H_5)_2$, to propionic acid was 1:2.

Examples 14 and 15 (comparison)

The method of Examples 1 to 9 was repeated using a heterogeneous catalyst system. The results are given in Table 2.

TABLE 2

| Example No. | Temp., °C | Catalyst components | Conversion of anhydride to products, % | Molar ratio acetic acid to ethylidene diacetate |
|---|---|---|---|---|
| 14 | 90 | Pd/Charcoal (1 g, 5 %w Pd)<br>p-toluene sulphonic acid (16 mmol) | 5 | 10 |
| 15 | 135 | Pd/charcoal (1 g, 5 %w Pd)<br>p-toluene sulphonic acid (16 mmol) | 15 | 8.4 |

These results show that the process as described in UK Application No. 2,034,307 cannot be carried out in the presence of carbon monoxide. When Example No. 14 was repeated using pure hydrogen, good yields of the desired products were obtained as described in said Specification.

Example 16 (comparison)

The method of Examples 1 to 9 was repeated. The catalyst was $RhCl_3.3H_2O$ (1 mmol), triphenylphosphine (2.5 mmol) and p-toluene sulphonic acid (16 mmol). When the reaction was carried out at 135°C, the conversion of anhydride was high, 60%, but the ratio of acetic acid to ethylidene diacetate was 5.0:1. In addition, 20 %m of other products were obtained. When the reaction was carreid out at 90°C, the conversion was only 10% and the molar ratio of acetic acid to ethylidene diacetate was 2.1:1.

Example 17

Preparation of acetic anhydride

A Hastelloy C 300 ml autoclave was charged with 150 ml methyl acetate, 1 mmol $RhCl_3.3H_2O$, 12 g lithium iodide hydrate and 3 g water. Carbon monoxide was added to a pressure of 30 bar, and the temperature was raised to 175°C and maintained at that level for 4 hours. Analysis of the reaction mixture showed that it contained 48% by weight of acetic anhydride and 13% by weight of acetic acid. This corresponds to a rate of production of acetic anhydride of 180 g per g Rh per hour.

Examples 18 to 21

Preparation of acetic anhydride

A Hastelloy C 300 ml autoclave was charged with 50 ml methyl acetate, 1 mmol $RhCl_3.3H_2O$, 8.5 g sodium iodide (which contains no water of crystallisation) and the other components listed in the table. The autoclave was pressurised to 30 bar with carbon monoxide. After 10 hours at 155°C, the contents were analysed. The results are given in Table 3.

TABLE 3

| Example No. | Additional components | Weight % acetic anhydride in product |
|---|---|---|
| 18 | None | 4.7 |
| 19 | 10 ml acetic acid | 25 |
| 20 | 2 mmol 15-crown-5 | 14.6 |
| 21 | 10 ml acetic acid<br>2 mmol 15-crown-5 | 58 |

**Claims**

1. A process for the co-production of an alkylidene dicarboxylate and a carboxylic acid, characterized in that a carboxylic acid anhydride is hydrogenated in the presence of carbon monoxide

and a homogeneous Group VIII metal catalyst together with a chloride, bromide or iodide in addition to any halide being present in the Group VIII metal catalyst and in the additional presence of a promotor comprising an organo oxygen, nitrogen, phosphorus, arsenic or antimony compound having a lone pair of electrons.

2. A process as claimed in claim 1, characterized in that the Group VIII metal is palladium, rhodium or nickel.

3. A process as claimed in claim 1 or 2, characterized in that the chloride, bromide or iodide is added to the reaction mixture in the form of an alkyl halide.

4. A process as claimed in claim 3, characterized in that the alkyl halide is methyl iodide.

5. A process as claimed in claims 1 to 4, characterized in that the promotor is a tri-organo phosphine.

6. A process as claimed in any one of claims 1 to 5, characterized in that the anhydride feedstock has been prepared in a separate reaction step by the carbonylation of an ester in the presence of a suitable catalyst.

7. A process as claimed in claim 6, characterized in that the ester has been carbonylated in the presence of a rhodium-containing catalyst together with a halide.

8. A process as claimed in either claim 6 or claim 7, characterized in that the ester has been carbonylated in the presence of a small quantity of water or a carboxylic acid.

9. A process as claimed in any one of claims 6 to 8, characterized in that the ester has been carbonylated in the presence of a metal halide promotor and a crown ether capable of complexing the metal ion.

10. A process as claimed in any one of claims 6 to 9, characterized in that the ester has been carbonylated using a stream of carbon monoxide which has been produced by carrying out a process as claimed in any one of claims 1 to 5 using as input a mixed carbon monoxide/hydrogen stream.

**Patentansprüche**

1. Verfahren zur gleichzeitigen Herstellung eines Alkylidendicarbonxylats und einer Carbonsäure, dadurch gekennzeichnet, daß ein Carbonsäureanhydrid in Gegenwart von Kohlenmonoxid und einem homogenen Katalysator auf der Basis eines Metalls der Gruppe VIII zusammen mit einem Chlorid, Bromid oder Iodid zusätzlich zu gegebenenfalls im Katalysator auf der Basis des Metalls der Gruppe VIII vorhandenem Halogenid und zusätzlich in Gegenwart eines Promotors, umfassend eine organische Sauerstoff-, Stickstoff-, Phosphor-, Arsen- oder Antimonverbindung mit einem einsamen Elektronenpaar hydriert wird.

2. Verfahren nach Anspruch 1, dadurch gekennzeichnet, daß das Metall der Gruppe VIII Palladium, Rhodium oder Nickel ist.

3. Verfahren nach Anspruch 1 oder 2, dadurch gekennzeichnet, daß das Chlorid, Bromid oder Iodid dem Reaktionsgemisch in Form eines Alkylhalogenids zugesetzt wird.

4. Verfahren nach Anspruch 3, dadurch gekennzeichnet, daß das Alkylhalogenid Methyliodid ist.

5. Verfahren nach Anspruch 1 bis 4, dadurch gekennzeichnet, daß der Promotor ein Tri-organophosphin ist.

6. Verfahren nach einem der Ansprüche 1 bis 5, dadurch gekennzeichnet, daß das Anhydrid-Ausgangsmaterial in einer getrennten Reaktionsstufe durch Carbonylieren eines Esters in Gegenwart eines geeigneten Katalysators hergestellt worden ist.

7. Verfahren nach Anspruch 6, dadurch gekennzeichnet, daß der Ester in Gegenwart eines Rhodium enthaltenden Katalysators zusammen mit einem Halogenid carbonyliert worden ist.

8. Verfahren nach entweder Anspruch 6 oder Anspruch 7, dadurch gekennzeichnet, daß der Ester in Gegenwart einer kleinen Menge wasser oder einer Carbonsäure carbonyliert worden ist.

9. Verfahren nach einem Ansprüche 6 bis 8, dadurch gekennzeichnet, daß der Ester in Gegenwart eines Metallhalogenid-Promotors und eines Kronenethers, der das Metallion komplex binden kann, carbonyliert worden ist.

10. Verfahren nach einem der Ansprüche 6 bis 9, dadurch gekennzeichnet, daß der Ester unter Verwendung eines Kohlenmonoxidstroms carbonyliert worden ist, der durch Ausführen des Verfahrens nach einem der Ansprüche 1 bis 5 unter Verwendung eines gemischten Kohlenmonoxid/Wasserstoffstroms als Einspeisung hergestellt worden ist.

**Revendications**

1. Un procédé pour la coproduction d'un alcoylidène dicarboxylate et d'un acide carboxylique, caractérisé en ce qu'un anhydride d'acide carboxylique est hydrogéné en présence d'oxyde de carbone et d'un catalyseur homogène à base d'un métal du groupe VIII et même temps que d'un chlorure, bromure ou iodure en plus de tout halogénure présent dans le catalyseur à base d'un métal du groupe VIII et en la présence supplémentaire d'un promoteur comprenant un composé organo-oxygène, azote, phosphore, arsenic ou antimoine ayant un doublet électronique libre.

2. Un procédé selon la revendication 1, caractérisé en ce que le métal du groupe VIII est du palladium, du rhodium ou du nickel.

3. Un procédé selon la revendication 1 ou 2, caractérisé en ce que le chlorure, bromure ou iodure est ajouté au mélange réactionnel sous la forme d'un halogénure d'alcoyle.

4. Un procédé selon la revendication 3, caractérisé en ce que l'halogénure d'alcoyle est de l'iodure de méthyle.

5. Un procédé selon les revendications 1 à 4, caractérisé en ce que le promoteur est une triorgano phosphine.

6. Un procédé selon l'une quelconque des revendications 1 à 5, caractérisé en ce que la charge de départ d'anhydride a été préparée dans une étape de réaction séparée par la carbonylation d'un ester en présence d'un catalyseur approprié.

7. Un procédé selon la revendication 6, caractérisé en ce que l'ester a été carbonylé en présence d'un catalyseur contenant du rhodium en même temps que d'un halogénure.

8. Un procédé selon la revendication 6 ou la revendication 7, caractérisé en ce que l'ester a été carbonylé en présence d'une petite quantité d'eau ou d'un acide carboxylique.

9. Un procédé selon l'une quelconque des revendications 6 à 8, caractérisé en ce que l'ester à été carbonylé en présence d'un promoteur halogénure de métal et d'un éther couronne capable de complexer l'ion de métal.

10. Un procédé selon l'une quelconque des revendications 6 à 9, caractérisé en ce que l'ester a été carbonylé en utilisant un courant d'oxyde de carbone qui a été produit par mise en oeuvre d'un procédé selon l'une quelconque des revendications 1 à 5 en utilisant un courant mixte oxyde de carbone/hydrogène.